# EUROPÄISCHE PATENTSCHRIFT

(11) **EP 2 421 574 B1**
(45) Veröffentlichungstag und Bekanntmachung des Hinweises auf die Patenterteilung: **25.03.2020**
(21) Anmeldenummer: 10714197.0
(22) Anmeldetag: 14.04.2010
(51) Int. Cl.: A61M 1/00, A61M 1/36, A61M 5/165

(54) **GERINNSELFÄNGER, EXTERNE FUNKTIONSEINRICHTUNG, BLUTKREISLAUF SOWIE BEHANDLUNGSVORRICHTUNG**
BLOOD CLOT CATCHER, EXTERNAL FUNCTIONAL DEVICE, BLOOD CIRCULATION, AND TREATMENT DEVICE
PIÈGE À CAILLOTS, DISPOSITIF FONCTIONNEL EXTERNE, CIRCUIT SANGUIN ET DISPOSITIF DE TRAITEMENT

(30) Priorität: 23.04.2009 DE 102009018664; 10.06.2009 DE 102009024495; 10.06.2009 US 185623 P
(43) Veröffentlichungstag der Anmeldung: 29.02.2012
(73) Patentinhaber: Fresenius Medical Care Deutschland GmbH, 61352 Bad Homburg v.d.H. (DE)
(72) Erfinder: BRÜCKNER, Christoph, 97505 Geldersheim (DE); LAUER, Martin, 66606 St. Wendel (DE); WEIS, Manfred, 66606 St. Wendel (DE)
(74) Vertreter: Bobbert & Partner Patentanwälte PartmbB
(86) Internationale Anmeldenummer: PCT/EP2010/002297
(87) Internationale Veröffentlichungsnummer: WO 2010/121743

(56) Entgegenhaltungen:
- WO-A1-2008/065472
- DE-A1- 4 404 277
- DE-U1- 29 706 807
- DE-U1- 29 706 807
- US-A1- 2005 139 532
- US-B1- 6 468 225

## Beschreibung

Die vorliegende Erfindung betrifft eine Blutkassette gemäß dem Oberbegriff des Anspruchs 1.

Gerinnselfänger werden u. a. in extrakorporalen Blutkreisläufen eingesetzt, um Gerinnsel oder Thromben, welche im extrakorporalen Blutstrom vorliegen, aufzufangen bzw. zurückzuhalten.

Die US 2005/013952 A1 offenbart ein manifold assembly mit einem Filter.

Die WO 2008/065472 A1 offenbart einen zylinderförmigen Filter in einer Dialysekassette.

Die DE 44 04 277 A1 offenbart einen Blutfilter.

Die DE 297 06 807 U1 offenbart einen Vorfilter für eine Vorrichtung zum Sammeln von bei Operationen anfallendem Blut.

Die US 6,468,225 B1 offenbart Verfahren und Vorrichtungen um Knochengewebefragmente zu sammeln.

Eine Aufgabe der vorliegenden Erfindung ist, eine Blutkassette mit einem Gerinnselfänger anzugeben.

Diese Aufgabe wird durch eine Blutkassette mit den Merkmalen des Anspruchs 1 gelöst.

Der Gerinnselfänger der erfindungsgemäßen Blutkassette weist eine Siebfläche bzw. eine Gerinnselfängerfläche auf, welche zum Auffangen von Gerinnseln eines die Siebfläche durchströmenden Fluids geeignet und vorgesehen ist.

Der Begriff "Siebfläche", wie er hierin verwendet wird, bezeichnet eine Einrichtung oder Komponente des Gerinnselfängers, welche ein Durchtreten des Gerinnsels durch den Gerinnselfänger verhindern soll.

Vorzugsweise ist die Siebfläche eine Einrichtung mit einer mechanisch wirksamen Sieb- bzw. Filterwirkung ohne hierauf beschränkt zu sein.

Die Siebfläche kann alternativ oder ergänzend ferner dazu geeignet und vorgesehen sein, Gerinnsel beispielsweise auf physikalische und/oder chemische Weise zurückzuhalten oder aufzulösen.

Die Siebfläche ist erfindungsgemäß scheibenförmig ausgestaltet.

Der Begriff "scheibenförmig", wie er hierin verwendet wird, bedeutet, dass die Siebfläche - vorzugsweise im Wesentlichen oder vollständig - in Form einer Scheibe ausgestaltet ist.

Vorteilhafte Weiterentwicklungen der vorliegenden Erfindung sind jeweils Gegenstand der Unteransprüche.

Im Folgenden gilt: Ein den Gerinnselfänger durchströmendes Fluid tritt - vollständig oder in Teilen hiervon - auf einer Einströmseite in den Gerinnselfänger ein, durchströmt die Siebfläche und tritt auf einer Abströmseite aus dem Gerinnselfänger aus. Unter Gerinnseln sind erfindungsgemäß z. B. Thromben, feste Verunreinigungen und dergleichen zu verstehen.

Der Begriff "Fluid", wie er hierin verwendet wird, schließt, ohne darauf beschränkt zu sein, Flüssigkeiten, wie medizinische Flüssigkeiten, z. B. Blut, Gase, Emulsionen, Suspensionen, Dispersionen und dergleichen sowie deren Mischungen ein.

Die Scheibenform der Siebfläche kann in jeder erfindungsgemäßen Ausführungsform uneben und/oder wellig, gekrümmt oder dergleichen sein. Sie kann alternativ eben - im Sinne von flach bzw. in einer Ebene liegend - ausgestaltet sein.

Die Scheibenform gibt eine Struktur an, welche in einer ersten Dimension (der Haupterstreckung) deutlich größer ist als in einer hierauf senkrechten Richtung.

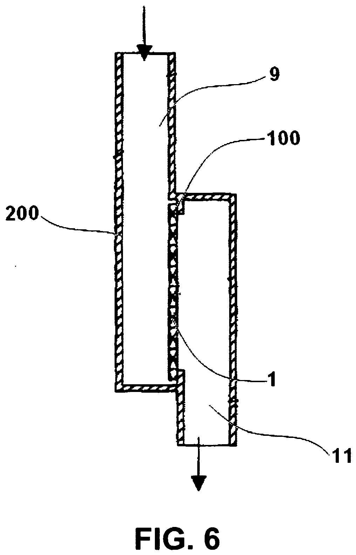

Der Gerinnselfänger ist während bzw. zu seiner Verwendung im Wesentlichen senkrecht - vorzugsweise bezogen auf die Haupterstreckung der Siebfläche - angeordnet, wie dies beispielsweise in Fig. 6 gezeigt ist.

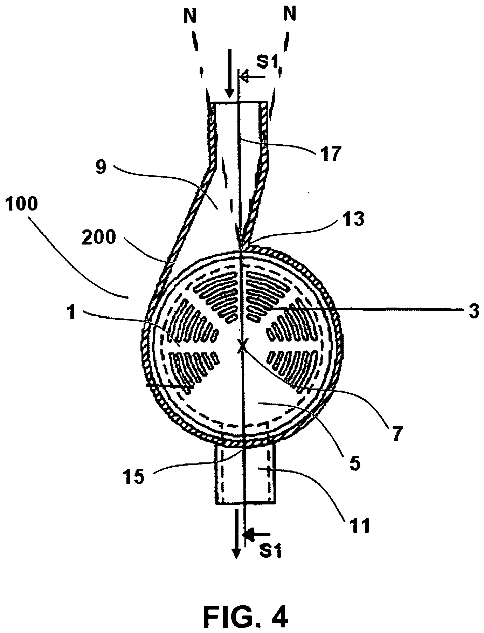

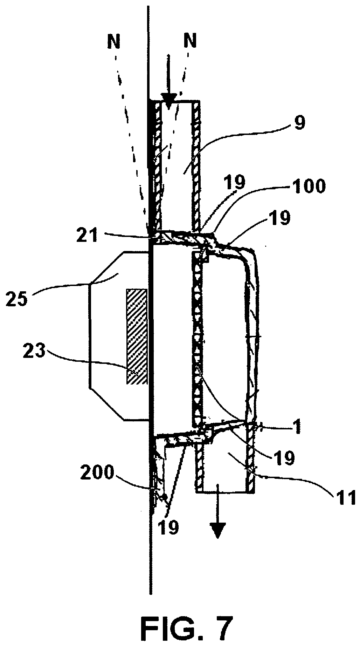

Der Begriff "im Wesentlichen senkrecht", wie er hierin verwendet wird, bedeutet, dass die Neigung des Gerinnselfängers gegen die Vertikale in allen Richtungen maximal etwa 15° betragen darf bzw. eine maximale Neigung von +/- 15° besonders bevorzugt ist. Eine Neigung um weniger als +/- 15° ist ebenfalls bevorzugt. Eine derartige Möglichkeit der Neigung um z. B. 15° im Uhrzeigersinn bzw. um 15° gegen den Uhrzeigersinn oder nach vorne bzw. nach hinten, oder zu einer Seite (also gegen jede Richtung) ist z. T. in den Fig. 4 und 7 angedeutet.

Die Begriffe "senkrecht" bzw. "vertikal" und "waagrecht" bzw. "horizontal" beziehen sich vorzugsweise auf den Erdmittelpunkt.

Der Gerinnselfänger kann im Gebrauch in einem beliebigen Winkel bzw. in einer beliebigen Schräge, bezogen auf den Erdmittelpunkt, angeordnet sein.

Eine chemische Filter- bzw. Siebwirkung kann erreicht werden, indem die Gerinnsel chemisch an die Siebfläche und/oder in der Siebfläche, beispielsweise in den Poren derselben, gebunden werden. Die Gerinnsel können reversibel oder irreversibel an die Siebfläche gebunden werden. Die Gerinnsel können physikalisch an der Siebfläche adsorbiert werden. Die Gerinnsel können ferner an der Siebfläche ganz oder teilweise aufgelöst werden.

Erfindungsgemäß weist die Siebfläche Durchtrittsöffnungen auf, durch welche das Fluid von der Einströmseite zur Abströmseite strömen kann. Mit Hilfe der Durchtrittsöffnungen kann eine mechanische Filter- bzw. Siebwirkung erreicht werden. Die Abmessungen der Durchtrittsöffnungen oder eine Maschenweite der Siebfläche können derart ausgewählt werden, dass die Gerinnsel aufgrund ihrer Größe auf der Einströmseite der einströmenden Fluide zurückgehalten werden.

Die Durchtrittsöffnungen der Siebfläche bilden zusammen eine offene Gesamtdurchtrittsfläche der Siebfläche.

Die Abmessungen der Durchtrittsöffnungen können so gewählt sein, dass im strömenden Fluid bzw. Fluidstrom vorhandene Gerinnsel, Agglomerate, Feststoffteilchen und dergleichen aufgrund ihrer Größe auf der Einströmseite des Fluids aus dem Fluidstrom zurückgehalten werden und nicht auf die Abströmseite des Gerinnselfängers gelangen.

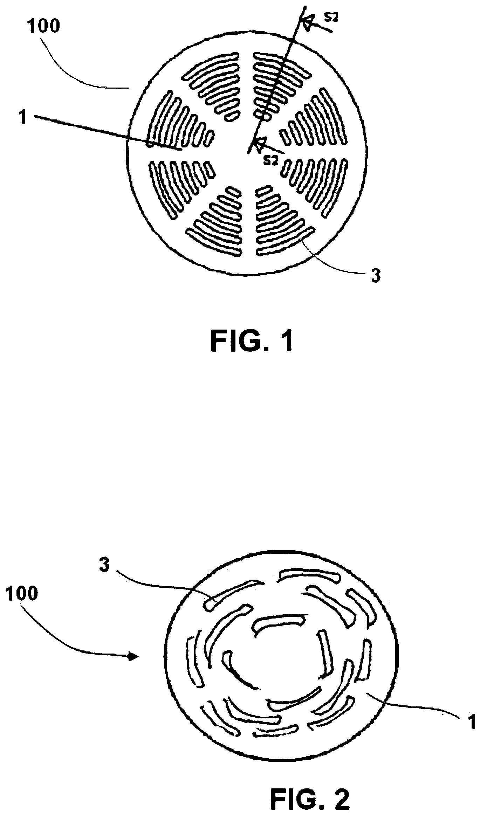

In einer weiteren bevorzugten Ausführungsform sind alle Durchtrittsöffnungen auf - insbesondere konzentrischen - Kreisen oder Kreisabschnitten angeordnet. Eine beispielhafte Anordnung ist nachfolgend in Fig. 1 gezeigt.

Alternativ dazu ist lediglich eine Vielzahl von Durchtrittsöffnungen auf - insbesondere konzentrischen - Kreisen angeordnet.

In einer weiter bevorzugten Ausführungsform sind - alle oder nur einige der - Durchtrittsöffnungen radial, bezogen auf einen Mittelpunkt des Gerinnselfängers oder der Siebfläche, angeordnet.

Der Begriff "Mittelpunkt" bezeichnet einen Mittelpunkt oder einen Punkt in einem mittleren Bereich der scheibenförmigen Siebfläche und/oder des Gerinnselfängers. Dieser Mittelpunkt kann dem Schwerpunkt oder geometrischen Schwerpunkt der Siebfläche und/oder des Gerinnselfängers und/oder einem Kreismittelpunkt einer runden Siebfläche und/oder des Gerinnselfängers entsprechen.

Im Vergleich zu einer konzentrischen Anordnung der Durchtrittsöffnungen sind die Durchtrittsöffnungen in einer "radialen" Anordnung derart vorgesehen, dass sie sich vom Mittelpunkt der Siebfläche oder des Gerinnselfängers aus in radialer Richtung, insbesondere in einer oder mehreren Ebenen, welche parallel zu einer Haupterstreckungsebene der Siebfläche liegen, erstrecken.

In einer bevorzugten Ausführungsform ist eine Anzahl von Durchtrittsöffnungen auf der gesamten Siebfläche angeordnet.

Die Durchtrittöffnungen können in jeweils gleichem Abstand zueinander über die Siebfläche verteilt angeordnet sein. Die Durchtrittsöffnungen können zueinander versetzt angeordnet sein, wie dies beispielsweise in Fig. 2 gezeigt ist.

Die Durchtrittsöffnungen können symmetrisch, bezogen auf den Mittelpunkt der Siebfläche und/oder des Gerinnselfängers, auf der Siebfläche angeordnet sein. Sie können jedoch auch asymmetrisch auf der Siebfläche angeordnet sein.

In einer weiter bevorzugten Ausführungsform weist wenigstens ein Abschnitt der Siebfläche keine - oder verglichen mit anderen Bereichen der Siebfläche eine geringere Anzahl an - Durchtrittsöffnungen auf.

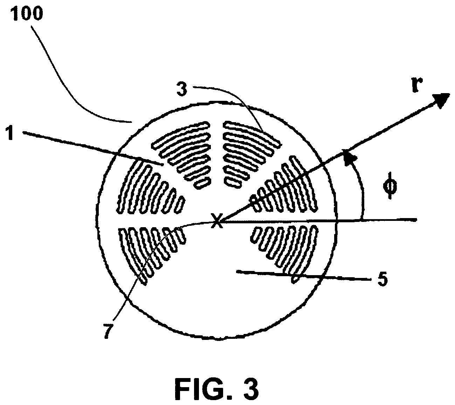

Ein Beispiel für eine derartige Anordnung ist in Fig. 3 und Fig. 4 gezeigt.

Der Abschnitt ohne - oder mit vergleichsweise nur wenigen - Durchtrittsöffnungen kann dem Abströmbereich des Gerinnselfängers oder eines Gehäuses, in welches der Gerinnselfänger aufgenommen ist, zugewandt sein.

Dieser Abschnitt kann die Form eines Kreissegments haben. Vorzugsweise liegt der Umfang des Teilkreisbogens stromabwärts zu einer Spitze des Teilkreisbogens in einer Gebrauchsstellung des Gerinnselfängers.

Die Durchtrittsöffnung(en) kann (können) eine beliebige geeignete geometrische Form aufweisen. Sie können beispielsweise eckig, rund, und/oder elliptisch oder dergleichen ausgestaltet sein.

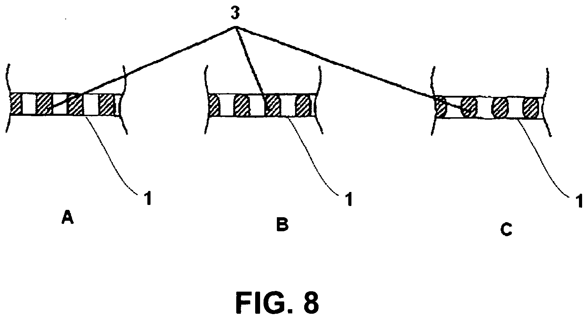

Eine Anzahl von Durchtrittsöffnungen kann beispielsweise auf genau einer der beiden Seiten der Siebfläche (Einströmseite und Abströmseite) scharfkantig ausgeführt sein, wie beispielsweise in Fig. 8A gezeigt ist.

Erfindungsgemäß ist in einer Alternative der Erfindung eine Anzahl von Durchtrittsöffnungen auf wenigstens einer Seite der Siebfläche abgerundet ausgeführt. Eine beispielhafte Ausführung ist in Fig. 8B gezeigt.

Die Durchtrittsöffnungen können in der anderen Alternative der Erfindung beidseitig verrundet ausgeführt sein. Eine beispielhafte Ausführung ist in Fig. 8C gezeigt.

Die Länge und/oder die Breite der Durchtrittsöffnungen kann beispielsweise in Abhängigkeit vom radialen Abstand bzw. von einem Abstand von einem mittleren Bereich ausgehend hin zum Rand der Siebfläche verändert werden. Beispielsweise kann die Länge und/oder die Breite der Durchtrittsöffnungen mit zunehmendem radialem Abstand nach außen hin ebenfalls zunehmen.

Die Länge und/oder die Breite der Durchtrittsöffnungen kann beispielsweise in Abhängigkeit von der Winkelorientierung verändert werden.

Ein "Winkel" kann ein Winkel eines Kreissegments zwischen einer Gerade durch den Mittelpunkt oder eines Punktes eines mittleren Bereichs des Gerinnselfängers oder der Siebfläche und einer anderen Gerade durch den Mittelpunkt der Siebfläche entlang des Kreisbogens des Gerinnselfängers oder der Siebfläche sein.

Der Gerinnselfänger kann wenigstens abschnittsweise aus einem hämokompatiblen Grundwerkstoff hergestellt sein. Der Gerinnselfänger kann vollständig aus einem hämokompatiblen Grundwerkstoff hergestellt sein.

Beispiele für geeignete hämokompatible Grundwerkstoffe schließen, ohne darauf beschränkt zu sein, PVP (Polyvinylpyrrolidon), welches vorzugsweise besonders hämokompatibel ist, sowie PP (Polypropylen) ein.

In einer weiter bevorzugten Ausführungsform ist der Gerinnselfänger aus einem Grundwerkstoff hergestellt. Geeignete Grundwerkstoffe schließen, ohne darauf beschränkt zu sein, Polypropylen, Polyethylen, Polycarbonat, Polyvinylchlorid (PVC), Polyamid (PA) und dergleichen ein.

Der Grundwerkstoff ist vorzugsweise mit einem oder mehreren hämokompatiblen Werkstoff(en) beschichtet.

Der Gerinnselfänger kann eine Spritzgusskomponente bzw. ein Spritzgusselement sein.

Die Siebfläche kann ein Siebgewebe sein. Sie kann im Wesentlichen oder vollständig als Gitterstruktur und/oder Maschenstruktur ausgestaltet sein. Die Siebfläche kann aus Filterpapier, Filtervlies oder dergleichen ausgestaltet oder hergestellt sein.

Die Siebfläche oder der Gerinnselfänger können durch eine Stützkonstruktion, wie ein Gehäuse, ein Gitter oder dergleichen, gestützt oder getragen werden. Eine Stützkonstruktion kann ebenfalls Sieb- bzw. Filterwirkung haben.

Beispielsweise kann ein stützendes Gitter an bzw. auf jeder Seite der Siebfläche vorgesehen sein.

In einer weiter bevorzugten Ausführungsform ist der Gerinnselfänger in einem Gehäuse angeordnet.

Ein Gehäuse kann zum Schutz des Gerinnselfängers bzw. der Siebfläche und/oder der Befestigung derselben an anderen Strukturen dienen.

In einer bevorzugten Ausführungsform ist der Gerinnselfänger mit dem Gehäuse verbunden.

Der Gerinnselfänger kann lösbar mit dem Gehäuse verbunden sein. Er kann beispielsweise mit dem Gehäuse verschnappt oder verrastet oder dergleichen sein.

Alternativ dazu kann der der Gerinnselfänger auch unlösbar mit dem Gehäuse verbunden sein. Er kann beispielsweise mit dem Gehäuse verschweißt oder verklebt oder dergleichen sein.

Das Verhältnis zwischen offener Gesamtdurchtrittsfläche und Gesamtfläche der Siebfläche oder Gesamtfläche des Gerinnselfängers kann vor Verwendung des Gerinnselfängers der Verwendung des Gerinnselfängers variiert werden.

Beispielsweise kann das Verhältnis zwischen offener Gesamtdurchtrittsfläche und Gesamtfläche der Siebfläche oder Gesamtfläche des Gerinnselfängers durch Ausgestalten bzw. Ausformen verschieden bzw. unterschiedlich großer Durchtrittsöffnungen, ggf. mit unterschiedlicher Form, und/oder Ausgestalten bzw. Anordnen oder Vorsehen des Abschnitts ohne Durchtrittsöffnungen bzw. Variieren der Größe und/oder Form desselben variiert werden.

Der Gerinnselfänger kann derart in ein Gehäuse integriert sein, dass er und/oder seine Siebfläche den Einströmbereich des Gehäuses vom Ausströmbereich trennen.

In einer weiteren bevorzugten Ausführungsform weist der Gerinnselfänger in wenigstens einem Abschnitt einer Oberseite desselben eine Abdeckungseinrichtung auf.

Die Abdeckungseinrichtung kann als Ankoppelfläche für wenigstens einen Sensor dienen, wie beispielsweise in Fig. 7 der beigefügten Zeichnung veranschaulicht ist. Sie kann hierzu vorbereitet sein. Sie kann entsprechende Einrichtungen zum Aufnehmen des Sensors oder eines hiermit bestückten Verbinders aufweisen wie Klemmen, Clipeinrichtungen und/oder dergleichen.

Alle Vorteile des Gerinnselfängers lassen sich ungeschmälert auch mit der erfindungsgemäßen Blutkassette erreichen, die wenigstens einen Gerinnselfänger wie hierin beschrieben aufweist.

Eine Blutkassette kann eine Einmalkomponente oder ein Einmalartikel sein. Sie kann aus einem Kunststoffmaterial gefertigt sein.

Die erfindungsgemäße Blutkassette kann zur Verwendung in einem Behandlungsverfahren angedacht sein. Behandlungsverfahren im Sinne der vorliegenden Erfindung schließen medizinische oder medizintechnische Behandlungsverfahren sowie Behandlungsverfahren der Labortechnik ein.

Eine Blutkassette, im Sinne der vorliegenden Erfindung ist beispielsweise in der von der Anmelderin der vorliegenden Erfindung in der beim DPMA am 23. April 2009 eingereichten Anmeldung 10 2009 018 664 A1 und in der am 10. Juni 2009 eingereichten Anmeldung 10 2009 024 468 A1, jeweils mit dem Titel *"Externe Funktionseinrichtung, Blutbehandlungsvorrichtung zum Aufnehmen einer erfindungsgemäßen externen Funktionseinrichtung, sowie Verfahren",* beschrieben.

Die Blutkassette kann zur Verwendung in oder an einer Behandlungsvorrichtung vorgesehen sein.

Die Behandlungsvorrichtung kann eine Blutbehandlungsvorrichtung, wie eine Dialysiervorrichtung zum Durchführen einer Dialysebehandlung, wie einer Hämodialyse, Hämofiltration, Hämodiafiltration und dergleichen, sein.

Im Vergleich zu herkömmlichen Gerinnselfängern, in welchen die aktive Siebfläche zylinderförmig oder kegelförmig ausgeführt ist, kann mit der durch den Gerinnselfänger der erfindungsgemäßen Blutkassette erzielbaren Strömungsführung ein Verstopfen des Gerinnselfängers bzw. der Siebfläche desselben vorteilhaft verstärkt verhindert werden.

Die scheibenförmige Ausgestaltung erlaubt ferner einen platzsparenden Einbau der Siebfläche innerhalb des Gerinnselfängers, gemessen beispielsweise an den eingangs erwähnten herkömmlichen zylinderförmigen Gerinnselfängern.

Die scheibenförmige Ausgestaltung kann ferner vorteilhafter Weise dazu beitragen, dass etwa eine Blutkassette oder ein Blutschlauch oder dergleichen, in welcher der Gerinnselfänger eingebaut wird, mit geringem Bauraumbedarf realisiert werden kann.

Insbesondere wenn der Gerinnselfänger mit einem Teilbereich ohne Durchtrittsöffnungen oder mit nur wenigen Durchtrittsöffnungen ausgeführt wird, kann ein Verschließen des Abströmbereichs auf der Rückseite des Gerinnselfängers bei potentiellem Wachstum eines mittels Gerinnselfängers aufgehaltenen Gerinnsels vorteilhaft verlangsamt werden.

Ferner kann durch Verändern der Länge und Breite der Durchtrittsöffnungen und/oder durch Variieren des Verhältnisses zwischen offener Gesamtdurchtrittsfläche und Gesamtfläche der Siebfläche oder Gesamtfläche des Gerinnselfängers die Durchströmung durch die Durchtrittsöffnungen vorteilhaft so verändert und optimiert werden, dass der Gerinnselfänger optimal durchströmt wird. Dazu kann auch die Gesamtdurchtrittsfläche der Durchtrittsöffnungen vorteilhaft optimiert werden. Eine optimierte Durchströmung kann zu verbesserten Strömungsverhältnissen führen und günstigen Einfluss u. a. auf ein weiteres Gerinnselwachstum am Gerinnselfänger haben.

Eine Verrundung der Durchtrittsöffnungen kann vorteilhaft dazu beitragen, eine Strömungsablösung der die Durchtrittsöffnungen durchströmenden Fluide wesentlich zu verringern oder zu verhindern.

Auf diese Weise kann es vorteilhaft möglich sein, eine Hämolyse und/oder eine Gerinnungsaktivierung des Blutes zu reduzieren.

Das Gehäuse, an welchem der Gerinnselfänger befestigt ist, kann in vorteilhafter Weise so gestaltet sein, dass Luftblasen, die sich auf der Einströmseite des Fluidstroms befinden, aufsteigen können. Die Luftblasen können so vorteilhaft den Einströmbereich des Gerinnselfängers verlassen. Dies ist insbesondere dann vorteilhaft möglich, wenn die Siebfläche des Gerinnselfängers mit ausreichender Steigung gegenüber einer Horizontalen ausgestaltet ist. Sie kann hierzu etwa 45 bis 90 Grad geneigt sein. Vorzugsweise ist sie senkrecht angeordnet.

Eine senkrechte oder im Wesentlichen senkrechte Anordnung der Siebfläche kann ferner in bestimmten Konstruktionen eine Platzeinsparung gegenüber einer horizontalen Anordnung bedeuten. Dies gilt insbesondere, wenn die senkrecht angeordnete Siebfläche parallel oder im Wesentlichen parallel zu einer Abdeckung (wie einer Folie) einer Blutkassette angeordnet ist, in welche sie integriert ist. Als Beispiel einer solchen Blutkassette sei wiederum die oben bereits genannte erwähnt.

Die Zuströmung zum Gerinnselfänger kann dabei in vorteilhafter Weise so erfolgen, dass die Einströmseite möglichst gut durchspült wird. Beispielsweise kann dies vorteilhaft mit einer möglichst tangentialen Zuströmung erreicht werden.

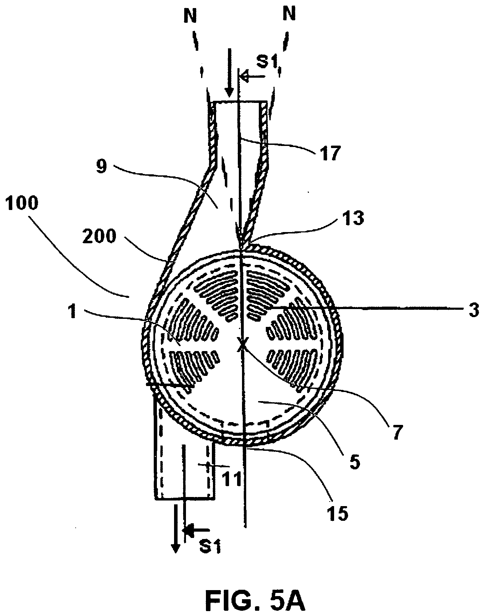

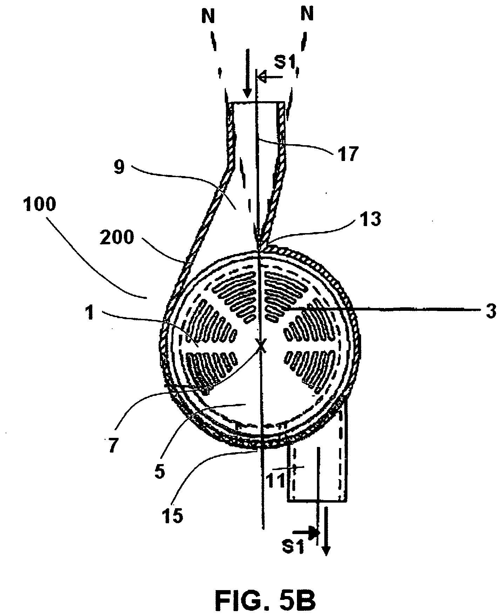

"Tangential" kann hierbei ein Anströmen des Gerinnselfängers im Wesentlichen in einer Ebene parallel zur Haupterstreckungsebene der Siebfläche bedeuten. Tangential kann wie in den Fig. 4, 5A und 5B sowie 6 zu erkennen verstanden werden. Gleichermaßen kann, wie in den Fig. 5A und 5B gezeigt, auch das Abströmen tangential oder seitlich oder in einem Randbereich des Gehäuses oder nicht mittig erfolgen.

Durch Anordnung der Durchtrittsöffnungen auf konzentrischen (Teil-)Kreisen kann zusätzlich vorteilhaft erreicht werden, dass sich eine Rotationsbewegung der Fluide - insbesondere bei tangentialer Ein- bzw. Zuströmung - auch auf die Abströmseite fortsetzt und so die Durchspülung der Abströmseite weiter verbessern kann.

Der vorliegende Gerinnselfänger kann somit vorteilhaft geeignet sein, der Bildung von Blutgerinnseln - im Sinne der Virchow'schen Trias - auf einfache oder mehrfache Weise entgegen zu wirken.

Im Folgenden wird die vorliegende Erfindung unter Bezugnahme auf die beigefügte Zeichnung beschrieben. In der Zeichnung bezeichnen identische Bezugszeichen gleiche oder identische Elemente. Es zeigt:
- Fig. 1: eine Frontansicht bzw. Ansicht von vorne auf die erfindungsgemäße Blutkassette mit einem Gerinnselfänger gemäß einer ersten Ausführungsform;
- Fig. 2: eine Front- bzw. Vorderansicht auf den Gerinnselfänger gemäß einer zweiten Ausführungsform;
- Fig. 3: eine Frontansicht auf den Gerinnselfänger gemäß einer dritten Ausführungsform;
- Fig. 4: eine Frontansicht auf den Gerinnselfänger der Fig. 3, der in einem ersten Gehäuse angeordnet ist;
- Fig. 5A: eine Frontansicht auf den Gerinnselfänger der Fig. 2 in einem zweiten Gehäuse;
- Fig. 5B: eine Frontansicht auf den Gerinnselfänger der Fig. 2 in einem dritten Gehäuse;
- Fig. 6: einen Längsschnitt des Gerinnselfängers aus Fig. 4;
- Fig. 7: einen Längsschnitt des Gerinnselfängers, welcher an einen Sensor angekoppelt ist; und
- Fig. 8A-C: Ausführungen von Durchtrittsöffnungen einer Siebfläche.

**Fig. 1** zeigt einen Gerinnselfänger 100 in einer erfindungsgemäßen Blutkassette gemäß einer ersten Ausführungsform mit einer Siebfläche 1 in einer Vorder- oder Frontansicht.

In der Siebfläche 1 sind Durchtrittsöffnungen 3 auf konzentrischen Teilkreisen angeordnet.

**Fig. 2** zeigt den Gerinnselfänger 100 gemäß einer zweiten Ausführungsform, in dessen Siebfläche 1 die Durchtrittsöffnungen 3 versetzt zueinander auf Kreisen - möglich sind auch Teilkreise - vorgesehen sind. Durch das Versetzen kann eine Überlappung in radialer Richtung entstehen. Dies ist jedoch nicht erforderlich.

**Fig. 3** zeigt einen Gerinnselfänger 100 gemäß einer dritten Ausführungsform.

Der Gerinnselfänger 100 weist in der Siebfläche 1 Durchtrittsöffnungen 3 auf, welche auf konzentrisch angeordneten Teilkreisen liegen. Die Teilkreise untergliedern die Siebfläche 1 in Segmente. Die Durchtrittsöffnungen 3 sind ebenfalls in Segmenten angeordnet.

In einem Abschnitt 5 sind keine Durchtrittsöffnungen vorgesehen.

Die Länge der Durchtrittsöffnungen 3 ändert sich wie schon in Fig. 1 in Abhängigkeit vom radialen Abstand r der Durchtrittsöffnungen 3 von einem Mittelpunkt 7 der Siebfläche 1.

Die Länge der Durchtrittsöffnungen 3 ändert sich in Umfangsrichtung der Siebfläche 1 in Abhängigkeit von der Winkelorientierung φ der Durchtrittsöffnungen 3 von einem Mittelpunkt 7 der Siebfläche 1.

**Fig. 4** zeigt eine Frontansicht auf den Gerinnselfänger 100 aus Fig. 3, welcher hier in einem ersten Gehäuse 200 angeordnet ist.

In einem Einströmbereich 9 des Gehäuses 200 ist ein tangentialer Zustrom - also auf einen Randbereich oder auf einen Umfang (welcher nicht als Stirnseite zu verstehen ist) der Siebfläche 1 gerichtet - eines Fluids vorgesehen. Der Fluidstrom bzw. die Fließ- oder Strömungsrichtung desselben sind hier und im Folgenden durch Pfeile gekennzeichnet.

Das Fluid bzw. der Fluidstrom tritt durch den Einströmbereich 9 in den Gerinnselfänger 100 ein und tritt an einem Abströmbereich 11 aus dem Gerinnselfänger 100 aus. Das Fluid tritt i. d. R. am höchsten Punkt 13 in den Gerinnselfänger ein und am tiefsten Punkt 15 aus dem Gerinnselfänger 100 aus. Die Lagebezeichnungen "höchster" Punkt 13 und "tiefster" Punkt 15 beziehen sich vorzugsweise auf eine Ausrichtung des Gerinnselfängers 100 während seiner Verwendung.

Wie mit den gestrichelten Linien N (Neigung) angedeutet ist, ist eine Neigung des Gerinnselfängers 100 während bzw. zu seiner Verwendung um - vorzugsweise - bis zu etwa 15°, bezogen auf eine Vertikale bzw. Senkrechte zum Erdmittelpunkt oder bezogen auf die Zeichnung, zulässig.

**Fig. 5A** zeigt eine Frontansicht bzw. Ansicht von vorne auf den Gerinnselfänger 100 in einem zweiten Gehäuse 200.

Im Gegensatz zum ersten Gehäuse 200, wie es z. B. in Fig. 4 gezeigt ist, ist der Abströmbereich 11 in Fig. 5A nach links versetzt zu einer Mittelachse 17, d. h. einer Achse durch den Mittelpunkt 7 des Gerinnselfängers, angeordnet.

Die Abströmung erfolgt tangential.

**Fig. 5B** zeigt eine Frontansicht auf den Gerinnselfänger in einem dritten Gehäuse 200.

Im Gegensatz zur Darstellung in Fig. 5A oder in Fig. 4 ist der Abströmbereich 11 - bezogen auf die Darstellung in Fig. 5B - nach rechts versetzt zu der Mittelachse 17 angeordnet.

Auch in der fünften Ausführungsform ist eine tangentiale Abströmung des den Gerinnselfänger durchströmenden Fluids vorgesehen.

**Fig. 6** zeigt einen Sagittal- oder Längsschnitt entlang S1-S1 aus Fig. 4 durch den Mittelpunkt 7. Die Linie S1-S1 entspricht, wie in Fig. 4 gezeigt, der Mittelachse 17 durch ein Zentrum des Gerinnselfängers 1.

Der Gerinnselfänger 100 ist senkrecht im Gehäuse 200 angeordnet.

Der Abschnitt 5 ohne Durchtrittsöffnungen liegt in Fig. 6 unten, dem Abströmbereich 11 zugewandt oder diesem benachbart.

**Fig. 7** zeigt einen Längsschnitt des Gerinnselfängers 100 in einem wiederum unterschiedlich ausgestalteten Gehäuse 200.

Das Gehäuse 200 weist, wie in Fig. 7 gezeigt ist, Abstufungen bzw. Schrägen 19 auf.

Der Gerinnselfänger 100 ist mit einer Abdeckungseinrichtung, z. B. einer Folie 21 versehen.

Die Folie 21 kann als Ankoppelfläche für einen Sensor 23 über dem Gerinnselfänger 100 dienen. Der Sensor 23 kann ein Drucksensor sein. Mittels des Sensors 23 kann beispielsweise ein Druck eines im Einströmbereich 9 des Gerinnselfängers 100 befindlichen Fluids gemessen werden.

Der Sensor 23 ist über einen Konnektor oder Verbinder 25 mit beispielsweise einer Behandlungsvorrichtung (nicht gezeigt) bzw. einer Steuer- oder Regeleinrichtung (nicht gezeigt) derselben verbunden.

Auch in Fig. 7 deuten die gestrichelten Linien N (Neigung) an, dass der Gerinnselfänger 100 während bzw. zu seiner Verwendung um - vorzugsweise - bis zu etwa 15°, bezogen auf eine Vertikale bzw. Senkrechte zum Mittelpunkt, geneigt sein kann.

Die **Fig. 8A bis 8C** zeigen mögliche Ausführungen von Durchtrittsöffnungen 3 der Siebfläche 1.

Die Fig. 8A-C zeigen Ausgestaltungen der Durchtrittsöffnungen 3 entlang eines Schnitts S2S2 durch die Siebfläche 1 gemäß Fig. 1.

Die Durchtrittsöffnungen 3 sind in Fig. 8A scharfkantig ausgeführt, was nicht der Erfindung entspricht.

Die Durchtrittsöffnungen 3 sind in Fig. 8B einseitig verrundet (in der Fig. 5B oben) ausgeführt.

In Fig. 8C sind die Durchtrittsöffnungen 3 beidseitig verrundet ausgeführt.

## Patentansprüche

1. Blutkassette, welche einen Gerinnselfänger (100) aufweist,
wobei der Gerinnselfänger (100) eine Siebfläche (1) zum Auffangen von Gerinnseln in einem die Sieb fläche (1) durchströmenden Fluid aufweist, wobei die Siebfläche (1) scheibenförmig ausgestaltet ist und Durchtrittsöffnungen (3) aufweist, durch welche das Fluid von einer Einströmseite zu einer Abströmseite der Siebfläche (1) strömen kann,
**dadurch gekennzeichnet, dass**
eine Anzahl der Durchtrittsöffnungen (3) auf wenigstens einer Seite der Siebfläche (1) abgerundet oder beidseits verrundet ausgeführt sind.

2. Blutkassette nach Anspruch 1, wobei im Gerinnselfänger (100) eine Anzahl der Durchtrittsöffnungen (3) auf konzentrischen Kreisen oder Teilkreisen angeordnet sind.

3. Blutkassette nach einem der vorangegangenen Ansprüche, wobei im Gerinnselfänger (100) eine Anzahl der Durchtrittsöffnungen (3) radial, bezogen auf einen Mittelpunkt (7) des Gerinnselfängers (100) oder der Siebfläche (1), angeordnet sind.

4. Blutkassette nach einem der vorangegangenen Ansprüche, wobei im Gerinnselfänger (100) eine Anzahl der Durchtrittsöffnungen (3) auf der gesamten Sieb fläche (1) angeordnet ist.

5. Blutkassette nach einem der Ansprüche 2 bis 4, wobei im Gerinnselfänger (100) ein Abschnitt (5) der Siebfläche (1) keine Durchtrittsöffnungen (3) aufweist.

6. Blutkassette nach Anspruch 5, wobei der Abschnitt (5) einem Abströmbereich (11) des Gerinnselfängers (100) oder eines Gehäuses (200), in welches der Gerinnselfänger (100) aufgenommen ist, zugewandt ist.

7. Blutkassette nach einem der vorangegangenen Ansprüche, wobei eine Anzahl der Durchtrittsöffnungen (3) auf einer der beiden Seiten der Siebfläche (1) scharfkantig ausgeführt ist.

8. Blutkassette nach einem der vorangegangenen Ansprüche, wobei der Gerinnselfänger (100) mit wenigstens einem hämokompatiblen Werkstoff beschichtet ist.

9. Blutkassette nach einem der vorangegangenen Ansprüche, wobei der Gerinnselfänger (100) in einem Gehäuse (200) angeordnet ist.

10. Blutkassette nach Anspruch 9, wobei der Gerinnselfänger (100) mit dem Gehäuse (200) verbunden ist.

11. Blutkassette nach Anspruch 9, wobei der Gerinnselfänger (100) mit dem Gehäuse (200) verschnappt ist.

12. Blutkassette nach Anspruch 9, wobei der Gerinnselfänger (100) mit dem Gehäuse (200) verschweißt ist.

13. Blutkassette nach einem der vorangegangenen Ansprüche, wobei der Gerinnselfänger (100) in wenigstens einem Abschnitt einer Oberseite desselben eine Abdeckungseinrichtung aufweist.

14. Blutkassette nach Anspruch 13, wobei die Abdeckungseinrichtung als Ankoppelfläche für wenigstens einen Sensor (23) dient.

15. Blutkassette nach Anspruch 14, wobei die Abdeckungseinrichtung eine Folie (21) ist.

## Claims

1. A blood cassette comprising a clot trap (100),
wherein the clot trap (100) comprises a sieve surface (1) for collecting clots in a fluid flowing through the sieve surface (1), the sieve surface (1) being disc-shaped and comprising passage openings (3) through which the fluid can flow from an inflow side to an outflow side of the sieve surface (1),
**characterized in that**
a number of the passage openings (3) is rounded on at least one side of the sieve surface (1) or rounded on both sides.

2. The blood cassette according to claim 1, wherein a number of the passage openings (3) is arranged on concentric circles or pitch circles in the clot trap (100).

3. The blood cassette according to anyone of the preceding claims, wherein a number of the passage openings (3) is arranged radially in relation to a center point (7) of the clot trap (100) or of the sieve surface (1) in the clot trap (100) .

4. The blood cassette according to anyone of the preceding claims, wherein a number of the passage openings (3) is arranged on the entire sieve surface (1) in the clot trap (100).

5. The blood cassette according to claims 2 to 4, wherein a portion (5) of the sieve surface (1) does not comprise any passage openings (3) in the clot trap (100).

6. The blood cassette according to claim 5, wherein the portion (5) faces an outflow region (11) of the clot trap (100) or of a housing (200) in which the clot trap (100) is received.

7. The blood cassette according to anyone of the preceding claims, wherein a number of the passage openings (3) on one of the two sides of the sieve surface (1) is sharp-edged.

8. The blood cassette according to anyone of the preceding claims, wherein the clot trap (100) is coated with at least one hemocompatible material.

9. The blood cassette according to anyone of the preceding claims, wherein the clot trap (100) is arranged in a housing (200) .

10. The blood cassette according to claim 9, wherein the clot trap (100) is connected with the housing (200).

11. The blood cassette according to claim 9, wherein the clot trap (100) is snapped into the housing (200).

12. The blood cassette according to claim 9, wherein the clot trap (100) is welded to the housing (200).

13. The blood cassette according to anyone of the preceding claims, wherein the clop trap (100) comprises a covering device in at least one portion of an upper side thereof.

14. The blood cassette according to claim 13, wherein the covering device serves as a coupling surface for at least one sensor (23).

15. The blood cassette according to claim 14, wherein the covering device is a film (21).

## Revendications

1. Une cassette à sang comprenant un piège à caillots (100),
où le piège à caillots (100) comprend une surface de tamisage (1) pour recueillir des caillots dans un fluide s'écoulant au travers de la surface de tamisage, (1), la surface de tamisage (1) étant conçue sous la forme d'un disque et comprenant des ouvertures de passage (3) au travers desquelles le fluide peut s'écouler d'une face d'entrée vers une face de sortie de la surface de tamisage (1),
**caractérisé en ce**
**qu'**un certain nombre d'ouvertures de passage (3) est arrondi sur au moins une face de la surface de tamisage (1) ou arrondi des deux côtés.

2. La cassette à sang selon la première revendication, où, dans le piège à caillots (100), un certain nombre d'ouvertures de passage (3) est agencé sur des cercles concentriques ou sur des cercles partiels.

3. La cassette à sang selon l'une quelconque des revendications précédentes, où, dans le piège à caillots (100), un certain nombre d'ouvertures de passage (3) est agencé radialement par rapport à un point central (7) du piège à caillots (100) ou à la surface de tamisage (1).

4. La cassette à sang selon l'une quelconque des revendications précédentes, où, dans le piège à caillots (100), un certain nombre d'ouvertures de passage (3) est agencé sur toute la surface de tamisage (1).

5. La cassette à sang selon l'une quelconque des revendications 2 à 4, où, dans le piège à caillots (100), une section (5) de la surface de tamisage (1) ne présente aucune ouverture de passage (3).

6. La cassette à sang selon la revendication 5, où la section (5) fait face à une zone de sortie (11) du piège à caillots (100) ou d'un boîtier (200) dans lequel le piège à caillots (100) est logé.

7. La cassette à sang selon l'une quelconque des revendications précédentes, où un certain nombre d'ouvertures de passage (3) est tranchant sur l'une des deux faces de la surface de tamisage (1).

8. La cassette de sang selon l'une quelconque des revendications précédentes, où le piège à caillots (100) est recouvert d'au moins un matériau hémocompatible.

9. La cassette à sang selon l'une quelconque des revendications précédentes, où le piège à caillots (100) est agencé dans un boîtier (200).

10. La cassette à sang selon la revendication 9, où le piège à caillots (100) est relié au boîtier (200).

11. La cassette selon la revendication 9, où le piège à caillots (100) est encliqueté dans le boîtier (200).

12. La cassette à sang selon la revendication 9, où le piège à caillots (100) est soudé au boîtier (200).

13. La cassette à sang selon l'une quelconque des revendications précédentes, où le piège à caillots (100) comprend un dispositif de recouvrement dans au moins une section d'une de ses faces supérieures.

14. La cassette à sang selon la revendication 13, où le dispositif de recouvrement sert de surface de couplage pour au moins un capteur (23).

15. La cassette à sang selon la revendication 14, où le dispositif de recouvrement est un film (21).
